# EUROPEAN PATENT APPLICATION

(11) **EP 4 269 624 A1**
(43) Date of publication of application: **01.11.2023**
(21) Application number: 21908244.3
(22) Date of filing: 07.01.2021
(51) Int. Cl.: C12Q 1/6888, C12Q 1/6883, C12Q 1/6809

(54) **MARKER GENE FOR IDENTIFYING AND CLASSIFYING DPC CELL, SCREENING METHOD THEREFOR AND USE THEREOF**

(30) Priority: 25.12.2020 CN 202011563849
(71) Applicant: Iregene Therapeutics Ltd, Wuhan, Hubei 430000 (CN)
(72) Inventor: CAI, Meng, Wuhan, Hubei 430000 (CN); WEI, Jun, Wuhan, Hubei 430000 (CN)
(74) Representative: Goddar, Heinz J.
(86) International application number: PCT/CN2021/070593
(87) International publication number: WO 2022/134225

(57) **Abstract**

Provided are a marker gene for identifying and classifying dopaminergic precursor cells (DPC), a screening method therefor and use thereof. The marker gene is a combination of 12 genes having specific up-regulation expression in the DPC cells relative to the neural stem cells (NSCs). The screening of the marker gene is based on a high-throughput RNA sequencing technology. In the samples of the NSC cells and the DPC cells, genes of which the specific expression is significantly up-regulated in the DPC cells are detected, and by bioinformatic analysis, a group of candidate genes capable of regulating the differentiation process of neural stem cells to dopaminergic neurons are identified. The marker gene combinations provided can be used to identify and classify DPC cell products for clinical treatment and to control the quality of the DPC cell products for clinical application.

## Description

### Background of the Invention

### Field of the Invention

The present invention relates to the field of biotechnology, in particular to marker gene for identifying and classifying dopaminergic precursor cells (DPC), a screening method therefor and use thereof.

### Description of Related Art

Parkinson's disease (PD) is a neurodegenerative disease with an incidence rate second only to that of Alzheimer's disease. It is common in the elderly, with an average age of about 60 years, and cannot be cured at present. The main pathological change of Parkinson's disease is the degeneration and death of dopaminergic neurons in the substantia nigra of the midbrain, which leads to a significant decrease in the DA content of the striatum and causes the disease. In clinical practice, the specific manifestations are motor symptoms, such as resting tremor, bradykinesia, rigidity, postural instability and gait disturbance, as well as non-motor symptoms, such as constipation, sleep disorders, and psycho-behavioral abnormalities. Due to the acceleration of population aging in China, as of the end of 2014, the population of elderly people aged 60 and above in China has reached 212 million, accounting for 15.5% of the total population. Among them, neurodegenerative diseases in the elderly, including Alzheimer's disease and Parkinson's disease, will become a major challenge for the Chinese medical system. According to statistics, the proportion of Parkinson's disease among people aged 60 and above in China is 1%, while the proportion of Parkinson's disease among people aged 65 and above has increased to 1.7% (Tian et al., 2011). Based on this calculation, the number of Parkinson's disease patients in China has reached 2.12 million, and it will reach 4 million in the future, making it the "third killer" of middle-aged and elderly people after tumors and cardiovascular and cerebrovascular diseases. Currently, Parkinson's patients are becoming younger, with early onset Parkinson's disease patients accounting for 10% of the total population.

Neurodegenerative diseases are originated from the aging and degeneration of nerve cells. It is generally believed that Parkinson's disease is caused by significant loss of dopaminergic neurons in the midbrain of patients. At present, drugs are mainly used to supplement or stimulate levodopa, which is insufficient in the brain. However, drug treatment has the disadvantages of many side effects and long-term decline in efficacy. After long-term medication, the sensitivity of neurons in the brain to drugs may decrease, leading to "dyskinesia" or "fluctuations in drug efficacy". In addition to drug therapy, there are two main clinical surgical treatments for Parkinson's disease: stereotactic ablation and deep brain stimulation (DBS) (Malek, 2019), but none of them can achieve good therapeutic effects. At present, the transplantation of dopaminergic progenitor cells (DPCs) derived from induced pluripotent stem cells (iPSC) to supplement and replace degenerated dopaminergic neurons has become a key research direction of stem cell regenerative medicine. However, DPC cells have a similar gene expression profile compared to neural stem cells, and it is usually difficult to distinguish DPC cells using the molecular markers of neural cells.

Therefore, mining a batch of gene markers with phenotypic differences in DPC and NSC cells for identification and classification of DPC cell products for clinical treatment is one of the key nodes in the quality control process of DPC cell clinical application.

### Summary of the Invention

The problem to be solved by the present invention is to provide marker genes for DPC cell identification and classification, a screening method, and an application for identifying and classifying DPC cell products for clinical treatment, and controlling the quality of DPC cell clinical products.

In view of this, the technical solution adopted to achieve the above purpose of the present invention is:
The application of marker genes in DPC cell identification and classification, wherein the marker genes are one or more of CRH, FST, GLI1, POSTN, PYCR1, RELN, SLC16A9, SLC7A2, ST8SIA3, SLC7A5, STK32B, FGF17.

Furthermore, in the above application, the DPC cell is identified and classified using RNA expressed by the marker gene, protein or biological precursor encoded by the marker gene.

Another object of the present invention is to provide a screening method for marker genes used for DPC cell identification and classification, comprising the following steps:
1. Extract and detect the total RNA of iPSC cells, NSC cells, and DPC cells respectively;
2. Construct a chain specific cDNA library and perform library analysis;
3. Sequencing and analyzing data to obtain genes with differences in RNA expression levels;
4. Based on the identified differentially expressed gene population, analyze the function of the genes and screen out genes that regulate neural cell function or are related to neural development and differentiation.

Furthermore, the marker genes obtained by the screening method are one or more of CRH, FST, GLI1, POSTN, PYCR1, RELN, SLC16A9, SLC7A2, ST8SIA3, SLC7A5, STK32B, FGF17.

Furthermore, the method for constructing a chain specific cDNA library in step S2 is:
A. Enrich eukaryotic mRNA and break into short fragments, use mRNA as a template to synthesize one strand cDNA with random primers, then synthesize two strand cDNA, and purify the double strand;
B. After end repair with A-tailing and connection to sequencing connector, select and extract fragment with correct size;
C. Degradation of the second strand of cDNA containing U;
D. PCR amplification, purification of the product, and obtaining a strand specific cDNA library.

Furthermore, the step S3 is to compare Clean reads with the reference genome sequence, calculate the number of reads (i.e. expression level) contained in each gene based on the annotation file of the genome and the alignment of reads to the location of the genome; Perform statistical testing to obtain differentially expressed genes; Finally perform functional annotation and GO, KEGG enrichment analysis on differentially expressed genes to obtain GO, KEGG sets enriched by differentially expressed genes.

Furthermore, the step S4 involves screening specific marker gene combinations for DPC cells using a 2.4-fold increase in expression level as a threshold, analyzing the functions of the selected genes, and selecting genes related to neural cell development and differentiation or neural cell function.

Another object of the present invention is to provide a detection kit, including primers designed based on marker genes or biological precursors of marker genes, wherein the marker genes are one or more of CRH, FST, GLI1, POSTN, PYCR1, RELN, SLC16A9, SLC7A2, ST8SIA3, SLC7A5, STK32B, FGF17.

Another object of the present invention is to provide the application of the reagent kit above in detecting DPC identification and classification of cell markers. The reagent kit can also be used for preparing diagnostic preparations for Parkinson's disease.

Furthermore, the detection kit comprises one or more sets of primers with a nucleotide sequence of SEQ ID No: 1-2, SEQ ID No: 3-4, SEQ ID No: 5-6, SEQ ID No: 7-8, SEQ ID No: 9-10, SEQ ID No: 11-12, SEQ ID No: 13-14, SEQ ID No: 15-16, SEQ ID No: 17-18, SEQ ID No: 19-20, SEQ ID No: 21-22, or SEQ ID No: 23-24.

Furthermore, the expression of the gene marker is detected by the reagent kit, and the detection method is one of fluorescence quantitative PCR method, digital PCR method, gene chip method, in situ hybridization method, or flow cytometry.

Furthermore, the gene chip method further includes a probe for nucleic acid sequence hybridization, wherein the nucleic acid sequence is designed based on the biological precursor of the marker gene or the marker gene.

### Beneficial effects:

Compared to the prior art, the beneficial effects of the present invention are:
1. The marker genes provided by the present invention can serve as marker for the identification and classification of DPC cells, and can also serve as detection standard for quality control in clinical grade DPC cell preparation, which has important application value.
2. The marker genes provided by the present invention can be used for preparing diagnostic preparations for Parkinson's disease, and the detection methods include fluorescence quantitative PCR, digital PCR, gene chip method, in situ hybridization or flow cytometry for detecting cells; The detection methods also include using digital PCR, gene chip, in situ hybridization, or flow cytometry to detect and encode the expression levels of genes and their biological precursors. The marker genes provided by the present invention have the advantage of wide applicability.

### Brief Description of the Figures

In order to provide a clearer explanation of the technical solution of the embodiments of the present invention, a brief introduction will be given to the accompanying drawings required in the embodiments. It should be understood that the following drawings only illustrate certain embodiments of the present invention, and therefore should not be regarded as limiting the scope. For ordinary technical personnel in the art, other relevant drawings can also be obtained based on these drawings without creative labor.
Fig. 1 is a specific experimental flowchart for the preparation of RNA samples in the selected marker genes of the present invention.
Fig. 2 is a flowchart for analyzing sequencing data in the screening of marker genes of the present invention.
Figures 3A and 3B are volcanic plots of the differential expression analysis of genes differentially expressed between samples in the selected marker genes of the present invention.
Figures 4A and 4B are schematic diagrams of the quantitative analysis results of the expression of marker genes in different cells using fluorescence quantitative PCR method.

### Detailed Description of the Invention

The purpose of the present invention is to screen a batch of genetic markers with potential application value for DPC cell identification and classification, for the quality control application of clinical grade DPC cell products. In order to achieve the above purpose, the invention first collects samples of induced pluripotent stem cells (iPSC, the original source cell of neural stem cells), NSC cells (precursor cell of DPC which differentiated from iPSC cells) and DPC cells (dopaminergic progenitor cells further differentiated from NSC cells for 24 hours), extracts total RNA in these cells by molecular biology methods, and screens candidate gene populations by high-throughput RNA sequencing combined with bioinformatics methods. Subsequently, a set of genes related to regulating neural cell function and differentiation was further screened through bioinformatics methods, and the differential expression of this set of genes was verified in different cell samples through real-time quantitative PCR. Finally, 12 related genes were identified, including CRH, FST, GLI1, POSTN, PYCR1, RELN, SLC16A9, SLC7A2, ST8SIA3, SLC7A5, STK32B, FGF17, etc. This group of genes can serve as markers for identifying and classifying DPC cells, and can also serve as a quality control detection standard for clinical grade DPC cell preparation, with important application value.

The content of the present invention includes providing one or more genes in CRH, FST, GLI1, POSTN, PYCR1, RELN, SLC16A9, SLC7A2, ST8SIA3, SLC7A5, STK32B, FGF17, as well as the application of their coding genes or biological precursors in the identification and classification of DPC cell products.

The content of the present invention includes providing a diagnostic preparation for Parkinson's disease, includes detecting cells using fluorescence quantitative PCR method, digital PCR method, gene chip method, in situ hybridization method or flow cytometry; and also includes using digital PCR, gene chip methods, in situ hybridization or flow cytometry to detect the expression levels of marker genes, their coding genes, and biological precursors; The marker is based on one or more of CRH, FST, GLI1, POSTN, PYCR1, RELN, SLC16A9, SLC7A2, ST8SIA3, SLC7A5, STK32B, and FGF17.

Fluorescence quantitative PCR is a method of labeling PCR products using specific probes labeled with fluorescent dyes or fluorescent markers, in order to achieve real-time online tracking of the process of product generation in PCR reactions. The fluorescence values of the products are analyzed using corresponding software to derive the initial concentration in the sample to be tested. By using fluorescence quantitative PCR technology, the production process of products in traditional PCR can be monitored in real-time, avoiding the decrease in accuracy of results after the PCR reaction reaches the plateau period. At the same time, absolute quantification is truly achieved, which can determine the template copy number in the sample. At present, the fluorescence quantitative PCR detection system is very mature. Compared to traditional PCR reactions, it has advantages such as fast response, good repeatability, high sensitivity, strong specificity, and clear results.

Digital PCR is a new method for nucleic acid detection and quantitative analysis, which can achieve absolute quantification and low abundance gene detection. The working principle of digital PCR is to divide DNA or cDNA samples into many separate and parallel PCR reactions, where a single molecule can be amplified one million times or more. The unique advantage of digital PCR is that it can be used to generate absolute counts of target molecules in samples without the need for standards or internal references.

Gene chips, also known as DNAmicroarrays, are generally divided into three main types: 1) nucleic acid probes or cDNA fragments are physically fixed to the surface of polymers such as nylon membranes or nitrocellulose membranes, and then hybridized with target genes labeled with isotopes, and detected using autoradiography technology. 2) Fix nucleic acid probes or cDNA fragment arrays on glass materials using dot sampling method, and then hybridize with fluorescent labeled target genes for detection by fluorescence signal intensity. 3) Directly synthesize oligonucleotide probe arrays on hard surfaces such as glass materials, and then hybridize with fluorescent labeled target genes for fluorescence signal detection. In recent years, gene chips, as a large-scale and high-throughput detection technology, have been widely used in the disease diagnosis.

In situ hybridization is the process of using specific labeled DNA or RNA nucleic acids of known sequences as probes to perform sequence complementary hybridization with nucleic acids in cell or tissue slices, in order to accurately and quantitatively locate specific nucleic acid sequences. In situ hybridization technology can detect and observe nucleic acids in their original positions in cells or tissues, but the sensitivity and specificity of the detection are not high.

The product that uses fluorescence quantitative PCR method to detect gene expression of CRH, FST, GLI1, POSTN, PYCR1, RELN, SLC16A9, SLC7A2, ST8SIA3, SLC7A5, STK32B, FGF17 and other genes in DPC cell products contains a pair of primers specifically amplifying the above genes; The gene chip, in situ hybridization includes probes that hybridize with nucleic acid sequences such as gene transcripts, their coding genes, biological precursors, etc.

The purpose of the present invention is to provide a fluorescent real-time quantitative PCR kit that can be used for detecting and identifying DPC cell products. The kit detects genes such as CRH, FST, GLI1, POSTN, PYCR1, RELN, SLC16A9, SLC7A2, ST8SIA3, SLC7A5, STK32B, FGF17, and their biological precursors, using specific upstream and downstream primers. The specific primer sequence is shown in Table 1 (primer number singular represents Forward primer, and double represents Reversed primer).

**Table 1**

| Gene | Primer number | Forward primer | Reversed primer |
|---|---|---|---|
| CRH | SEQ ID NO: 1-2 | GCAGCAGCAACAC AATGTTATTCG | TGGCATTCTCTTTG GCTCAGACC |
| FST | SEQ ID NO: 3-4 | GCTGCTGCTCTGCC AGTTCAT | TGTGTTGTCATTCA CGTCCTCCTC |
| GLI1 | SEQ ID NO: 5-6 | ACATCAACAGCGA GCACATCCA | CGAGGCGTGAGTAT GACTTCCG |
| POSTN | SEQ ID NO: 7-8 | CCATGTCATTGACC GTGTGCTTAC | GCCTCCAATATGTC CGATGTGATG |
| PYCR1 | SEQ ID NO: 9-10 | TTCACAGCAGCAG GCGTCTTG | CACAGCCAGGAAG AGCACATCAC |
| RELN | SEQ ID NO: 11-12 | TCCACTGTGAGAC AAGCGGTTAC | TCCTCCATCGGTAG AGTAGTCCAA |
| SLC16A9 | SEQ ID NO: 13-14 | AACAGTGACCATTA CGTGCCAGTA | CCACAATCAGCAAG CATCCATCC |
| SLC7A2 | SEQ ID NO: 15-16 | TGGTTGATGCCACT GCCATGATC | AGTCTAAGAGCCCA CCCAACAAGG |
| ST8SIA3 | SEQ ID NO: 17-18 | TGACAGTGGCATCA GAGTGAAGAG | AAGGTCCAGAATGA GTGGCAAGAA |
| SLC7A5 | SEQ ID NO: 19-20 | AGGAGGCAGTGGA CATCGTAGG | CAGAGCAGGAGGT TGGAACAGAAC |
| STK32B | SEQ ID NO: 21-22 | TCCTGGTCAATCTG TGGTACTCCT | CTGGCTTGATGTCT CTGTGGATGA |
| FGF17 | SEQ ID NO: 23-24 | CCTGGAGAGGAAC TGAGTGTCACC | GCAGACTGAGGGA GATCCGAGAAG |

The PCR reagent kit described in the present invention uses SYBR Green fluorescent dye, which is suitable for all types of fluorescent quantitative gene amplification instruments currently on the market. It has high sensitivity, fast and accurate quantification, stable performance, and good market prospects.

The components of the SYBR Green fluorescence quantitative PCR kit mentioned above include specific primers, internal reference primers, and a mixture of fluorescence quantitative PCR reaction components (SYBR Green et al.). The specific primers mentioned therein include upstream primers and downstream primers, and the internal reference primers are beta actin and GAPDH primers.

The present invention also provides a gene chip for DPC cell products, which includes probes for hybridization with nucleic acid sequences of genes such as CRH, FST, GLI1, POSTN, PYCR1, RELN, SLC16A9, SLC7A2, ST8SIA3, SLC7A5, STK32B, FGF17, and their coding genes, biological precursors, etc.

The following will further elaborate on the present invention in conjunction with specific embodiments, which is only intended to explain the present invention and cannot be understood as a limitation of the present invention. Ordinary technical personnel in this field can understand that various changes, modifications, substitutions, and variations can be made to these embodiments without departing from the principles and purposes of the present invention. The scope of the present invention is limited by the claims and their equivalents.

The experimental methods in the following embodiments that do not specify specific conditions are usually tested under conventional conditions or according to the conditions recommended by the manufacturer.

### Examples

### Example 1: RNA was extracted from iPSC cells, NSC cells and DPC cells, and prepared for high-throughput RNA sequencing

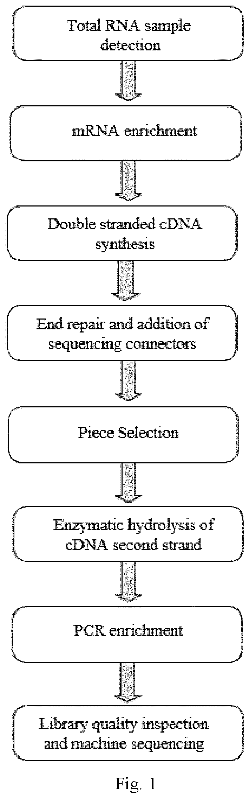

The specific experimental process for high-throughput RNA sequencing sample preparation is shown in Figure 1. The total RNAin iPSC cells (iPSC, the original source cell of neural stem cells), NSC cells (precursor cell of DPC which differentiated from iPSC cells for 24 hours) and DPC cells (dopaminergic progenitor cells further differentiated from NSC cells) was extracted by using the RNeasy MinElute Cleanup Kit (Cat No./ID: 74204) kit of Qiagen according to the requirements.

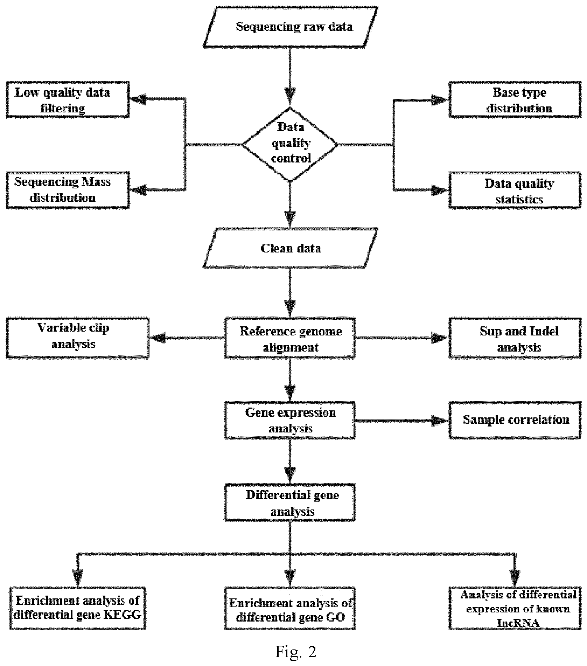

After extracting total RNA from the sample, the quality testing of the extracted total RNA sample mainly includes three methods:
1) Agarose gel electrophoresis was used to analyze the degradation degree of RNA and whether there was pollution;
2) Nanodrop detection of RNA purity (OD260/280 ratio);
3) Labchip accurately detects the integrity of RNA (as shown in Figure 2).

After passing the RNA sample quality detection, enrich eukaryotic mRNA with magnetic beads containing Oligo (dT) (in the case of prokaryotes, enrich mRNA by removing rRNA using a reagent kit). Subsequently, a fragmentation buffer was added to break the mRNA into short fragments. Using mRNA as a template, a single strand of cDNA was synthesized using six base random primers. Then, buffer, dNTPs, DNA polymerase I, and RNaseH were added to synthesize a double stranded cDNA. The double stranded cDNA was purified using AMPure XP beads. The purified double stranded cDNA is first subjected to end repair with an A-tailing and connected to a sequencing connector, followed by AMPure XP beads for fragment size selection. Afterwards, the second strand of cDNA containing U was degraded using the USER enzyme, resulting in the final sequencing information coming from the first strand of cDNA, thereby preserving the strand orientation of original mRNA. Finally, PCR amplification was performed and the PCR product was purified using AMPure XP beads to obtain a strand specific cDNA library.

After the construction of the library is completed, preliminary quantification is performed using Qubit3.0. Dilute the library to 1ng/uL, and then the insert size of the library is tested using Qsep100 instrument. After the insert size meets expectations, the effective concentration of the library is accurately quantified using Q-PCR method (effective concentration of the library>2nM) to ensure the quality of the library. After passing the library inspection, the different libraries were pooled according to the effective concentration and target offline data volume requirements, and then Illumina HiSeq sequencing was performed.

### Example 2: Analysis of data obtained after high-throughput RNA sequencing

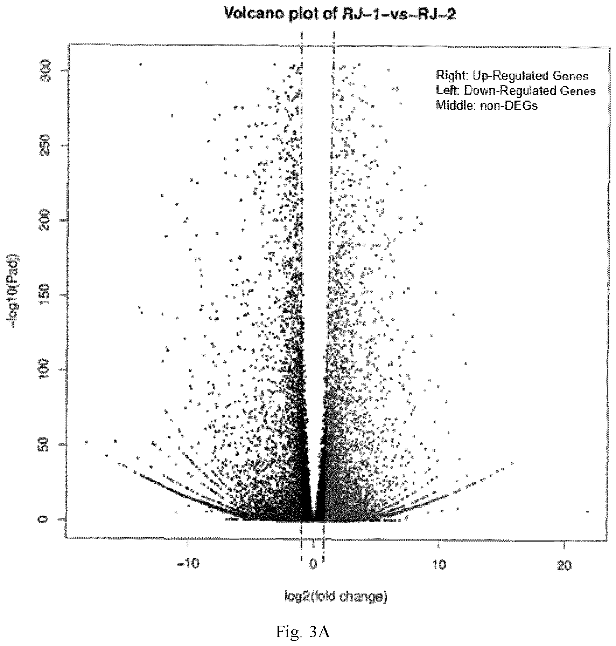

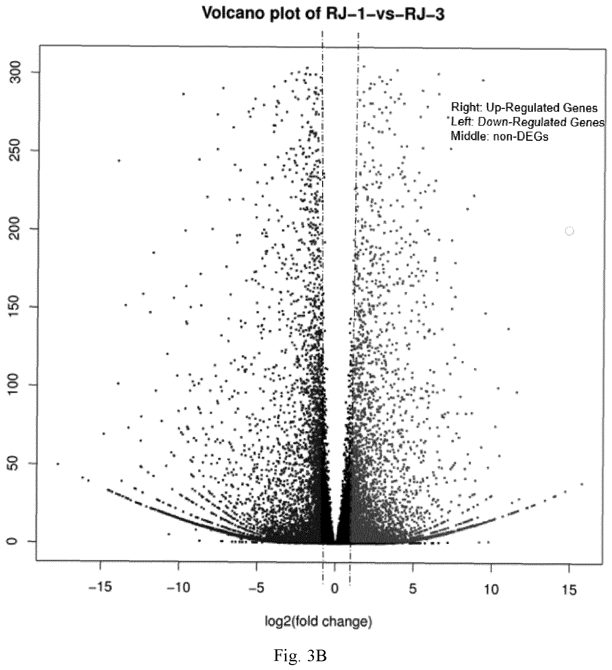

Filter the offline data to obtain Clean reads, compare Clean reads with the reference genome sequence, and calculate the number of reads contained in each gene based on the annotation file and reads of the genome. Then, perform statistical testing to obtain differentially expressed genes. Finally, perform functional annotation and GO, KEGG enrichment analysis on differentially expressed genes to obtain enriched differentially expressed gene sets. The overall process is shown in Figure 2. In specific analysis, RJ-1 (iPSC cells) was used as a standard parental cell to compare gene expression with RJ-2 (NSC cells) and RJ-3 (DPC cells), respectively. The differentially expressed genes analyzed are shown in volcano plots 3A and 3B in Figure 3. Each point in the differential expression volcano plot represents a gene, and the horizontal axis represents the logarithmic value of the multiple expression differences of a certain gene in two samples; The vertical axis represents the negative logarithmic value of the statistical significance of changes in gene expression. The larger the absolute value of the abscissa, the greater the difference in expression multiples between the two samples; The larger the ordinate value, the more significant the differential expression, and the more reliable the differentially expressed genes screened. For clarity, adjacent boundaries are divided by dashed lines running through the coordinate system in Figures 3A and 3B. The left point of the two dashed lines represents down-regulated genes, while the right point of the two dashed lines represents up-regulated genes. The two dashed lines are non-DEGs.

### Example 3: Analysis and screening of 12 up-regulated genes in DPC cells

Further analysis was conducted on the differentially expressed genes screened in Example 2 to identify specific marker gene combinations for DPC cells. Firstly, based on the analysis results in Example 2, we identified genes that were up-regulated in DPC cells relative to NSC cells. The range of these genes was screened using a 2.4-fold increase in expression as a threshold. Then, we used the Pubmed literature database on the NCBI website to analyze the functions of the selected genes, and selected genes related to neural cell development and differentiation or neural cell function. The results are shown in Table 2. Through the above analysis, a total of 12 genes were identified as candidate genes for specific marker of DPC cells, including CRH, FST, GLI1, POSTN, PYCR1, RELN, SLC16A9, SLC7A2, ST8SIA3, SLC7A5, STK32B, and FGF 17.

**Table 2**

| | RJ2 vs RJ1 | | | RJ3 vs RJ1 | | | RJ3 vs RJ2 | | | |
|---|---|---|---|---|---|---|---|---|---|---|
| Gene ID | Base mean | log2( FC) | P-adj | Base mean | log2( FC) | P-adj | Base mean | log2( FC) | P-adj | Gene name |
| ENSG000 00165449 | 544.6 565 | 2.024 475 | 5.39 E-87 | 1051. 47 | 3.321 183 | 0 | 1567. 745 | 1.285 815 | 2.53E-111 | SLC16A 9 |
| ENSG000 00189056 | 475.3 296 | 5.745 092 | 1.18 E-135 | 1032. 521 | 7.040 106 | 9.09 E-222 | 1678. 299 | 1.295 689 | 1.27E-106 | RELN |
| ENSG000 00003989 | 626.1 455 | - 5.652 04 | 1.96 E-224 | 585.6 565 | - 3.731 48 | 1.69 E-211 | 60.46 042 | 1.307 349 | 5.64E-10 | SLC7A2 |
| ENSG000 00133110 | 5.858 765 | - 0.094 66 | 0.923 076 | 21.11 284 | 2.635 271 | 2.53 E-07 | 24.37 19 | 1.352 491 | 4.22E-09 | POSTN |
| ENSG000 00158815 | 25.96 422 | 2.224 743 | 1.28 E-06 | 65.29 108 | 3.888 393 | 2.00 E-26 | 94.03 965 | 1.356 858 | 7.87E-14 | FGF17 |
| ENSG000 00152953 | 46.82 865 | - 0.594 15 | 0.063 505 | 82.78 177 | 1.191 397 | 6.55 E-07 | 86.61 068 | 1.423 572 | 1.26E-14 | STK32B |
| ENSG000 00177511 | 177.1 015 | 0.773 969 | 1.52 E-06 | 345.3 171 | 2.300 724 | 3.09 E-76 | 450.9 158 | 1.463 158 | 1.41E-52 | ST8SIA 3 |
| ENSG000 00183010 | 1340. 498 | - 2.593 38 | 6.85 E-283 | 1506. 696 | - 1.074 71 | 3.72 E-69 | 762.6 398 | 1.482 906 | 2.99E-85 | PYCR1 |
| ENSG000 00147571 | 21.65 966 | 5.532 145 | 1.06 E-09 | 68.29 075 | 7.140 733 | 5.01 E-19 | 101.6 414 | 1.504 652 | 1.13E-17 | CRH |
| ENSG000 00103257 | 3104. 478 | - 3.233 9 | 0 | 3290. 811 | - 1.645 85 | 1.36 E-286 | 1237. 188 | 1.565 839 | 5.26E-138 | SLC7A5 |
| ENSG000 00134363 | 120.6 474 | - 2.981 67 | 2.59 E-41 | 166.0 922 | - 0.424 83 | 0.016 532 | 96.27 073 | 2.009 824 | 2.01E-28 | FST |
| ENSG000 00111087 | 208.7 751 | - 7.769 26 | 1.95 E-45 | 220.2 895 | - 2.375 23 | 3.08 E-49 | 42.06 682 | 2.499 65 | 3.79E-30 | GLI1 |

### Example 4: Detect and confirm the expression levels of 12 highly expressed genes in DPC cells in iPSC cells , NSC cells and DPC cells one by one.

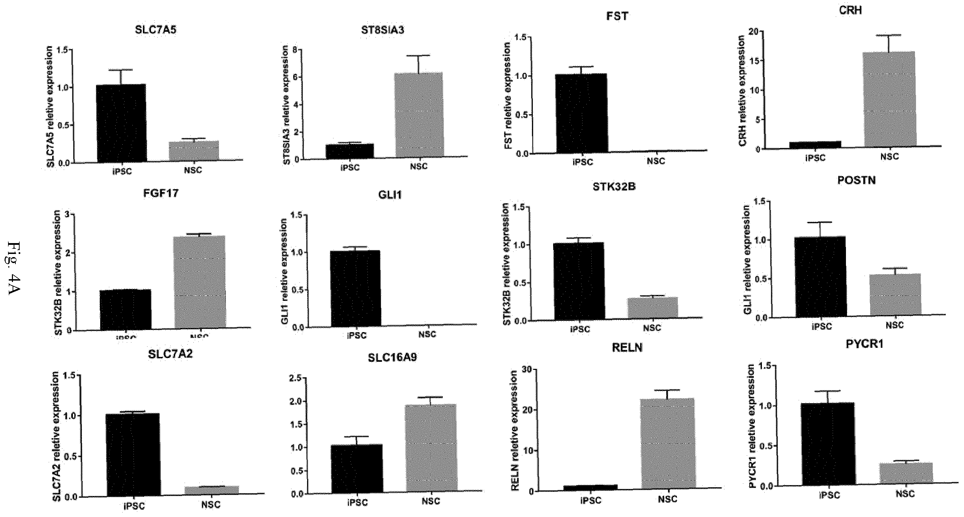

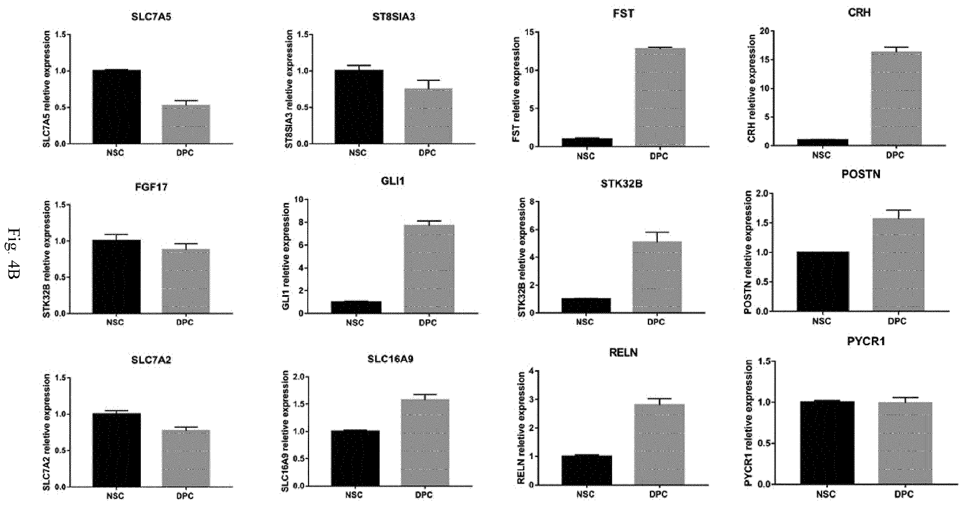

To further verify the accuracy of DPC specific expression genes screened by RNA high-throughput sequencing, we prepared different cell samples of iPSC cells (, the original source cell of neural stem cells), NSC cells (precursor cell of DPC which differentiated from iPSC cells) and DPC cells (dopaminergic progenitor cells further differentiated from NSC cells for 24 hours), extracted total RNA, and detected and compared the expression of the above 12 genes in these cells by fluorescence quantitative PCR. The expression levels of the above genes were analyzed and compared in iPSC cells and NSC cells, as well as NSC cells and DPC cells, as shown in Figures 4A and 4B.

### 4.1 Materials

Samples of iPSC cells, NSC cells, and DPC cells, approximately 5×10⁶ cells per sample.

### 4.2 Procedures and methods

### 4.2.1 Extraction of total RNA from Cells

Use the RNeasy MinElute Cleanup Kit (Cat No./ID: 74204) from Qiagen Company for total RNA extraction from the sample, and follow the product manual for operation. RNA quality criteria: the OD260/OD280 values of RNA samples are between 1.7 and 2.2.

### 4.2.2 Reverse transcription synthesis of cDNA

The standard reverse transcription method is employed, briefly described as follows:
Using a dedicated reverse transcription kit, approximately 1000 ng of total RNA was taken for reverse transcription to synthesize cDNA. The primers for reverse transcription use the special primers provided by the reagent kit.

### 4.2.3 Primer design

Using online primer design software, the primers for specific gene amplification were synthesized by Beijing Qingke Biotechnology Co., Ltd. The specific primer sequences are shown in Table 1.

### 4.2.3 Fluorescence quantitative PCR

PCR reaction was performed using Power Green PCR Master Mix (Invitrogen, article number 4367659), and followed the product manual for specific experimental operations. The amplification procedure is: 95° 10 min, (95°C 15 sec, 60°C 60 sec) × 45 cycles. Taking 2 µL - 5 µL cDNA from the sample as template, PCR amplification was performed using target gene specific primers and universal primers provided by the kit. The prepared reaction mix was subjected to relative quantitative analysis using the Quantagene q325 fluorescence quantitative PCR instrument and the 2-ΔΔCT method.

### 4.3 Experimental results

The relative quantitative and comparative analysis of the expression of 12 genes in different cell types by fluorescent quantitative PCR method confirmed that the expression of these 12 genes in iPSC cells and NSC cells conforms to the change trend of that in the RNA high-throughput sequencing. In Figure 4A that compared the gene expression in iPSC and NSC, the expression of ST8SIA3, CRH, FGF17, SLC16A9 and RELN gene in NSC cells was increased compared with iPSC cells, while the expression of SLC7A5, FST, GLI1, STK32B, POSTN, SLC7A2 and PYCR1 genes was decreased compared with iPSC cells. These results are consistent with the results of RNA high-throughput sequencing. In addition, as revealed in Figure 4A, comparing the gene expression in NSC and DPC cells, the expression trend of FST, CRH, GLI1, STK32B, POSTN, SLC16A9, RELN was consistent with that in RNA high-throughput sequencing, which was up-regulated in comparison to NSC cells, while SLC7A5, ST8SIA3, FGF17, SLC7A2, and PYCR1 showed no significant difference or slight decrease compared to NSC cells. Therefore, the results of gene expression determined by quantitative PCR confirmed the accuracy of RNA high-throughput sequencing.

It should be noted that the above embodiments are only the preferred specific embodiments of the present invention and do not limit the present invention in any form. Any technical solution obtained by equivalent replacement or modification based on the present invention is covered by the scope of protection of the present invention.

### Reference:

1.Tian YY,Tang CJ,Wu J,Zhou JS.Parkinson's disease in China.Neurol Sci.2011 Feb; 32(1):23-30.
2.Malek N.Deep Brain Stimulation in Parkinson's Disease.Neurol India.2019 Jul-Aug; 67(4):968-978.

## Claims

1. The application of marker genes in DPC cell identification and classification, wherein the marker genes are one or more of CRH, FST, GLI1, POSTN, PYCR1, RELN, SLC16A9, SLC7A2, ST8SIA3, SLC7A5, STK32B, FGF17.

2. The application of claim 1, wherein the DPC cell is identified and classified using RNA expressed by the marker gene, protein or biological precursor encoded by the marker gene.

3. A screening method for marker genes used for DPC cell identification and classification, comprising the following steps:
S1. Extract and detect the total RNA of iPSC cells, NSC cells, and DPC cells respectively;
S2. Construct a chain specific cDNA library and perform library analysis;
S3. Sequencing and analyzing data to obtain genes with differences in RNA expression levels;
S4. Based on the identified differentially expressed gene population, analyze the function of the genes and screen out genes that regulate neural cell function or are related to neural development and differentiation.

4. The screening method of claim 3, wherein the marker genes obtained by the screening method are one or more of CRH, FST, GLI1, POSTN, PYCR1, RELN, SLC16A9, SLC7A2, ST8SIA3, SLC7A5, STK32B, FGF17.

5. The screening method of claim 3, wherein the method for constructing a chain specific cDNA library in step S2 is:
1) Enrich eukaryotic mRNA and break into short fragments, use mRNA as a template to synthesize one strand cDNA with random primers, then synthesize two strand cDNA, and purify the double strand;
2) After end repair with A-tailing and connection to sequencing connector, select and extract fragment with correct size;
3) Degradation of the second strand of cDNA containing U;
4) PCR amplification, purification of the product, and obtaining a strand specific cDNA library.

6. The screening method of claim 3, wherein the step S3 is to compare Clean reads with the reference genome sequence, calculate the number of reads (i.e. expression level) contained in each gene based on the annotation file of the genome and the alignment of reads to the location of the genome; Perform statistical analysis to obtain differentially expressed genes; Finally perform functional annotation and GO, KEGG enrichment analysis on differentially expressed genes to obtain GO, KEGG-enriched differentially expressed genes.

7. The screening method of claim 3, wherein the step S4 involves screening specific marker gene combinations for DPC cells using a 2.4-fold increase in expression level as a threshold, analyzing the functions of the selected genes, and selecting genes related to neural cell development and differentiation or neural cell function.

8. A detection kit, including primers designed based on marker genes or biological precursors of marker genes, wherein the marker genes are one or more of CRH, FST, GLI1, POSTN, PYCR1, RELN, SLC16A9, SLC7A2, ST8SIA3, SLC7A5, STK32B, FGF17.

9. The reagent kit of claim 8, comprising one or more sets of primers with a nucleotide sequence of SEQ ID No: 1-2, SEQ ID No: 3-4, SEQ ID No: 5-6, SEQ ID No: 7-8, SEQ ID No: 9-10, SEQ ID No: 11-12, SEQ ID No: 13-14, SEQ ID No: 15-16, SEQ ID No: 17-18, SEQ ID No: 19-20, SEQ ID No: 21-22, or SEQ ID No: 23-24.

10. The application of the reagent kit of claim 8 in detecting DPC identification and classification of cell markers.

11. The application of the reagent kit of claim 8 in the preparation of diagnostic preparations for Parkinson's disease.

12. The application of claim 11, wherein the expression of the gene marker is detected by the reagent kit, and the detection method is one of fluorescence quantitative PCR method, digital PCR method, gene chip method, in situ hybridization method, or flow cytometry.

13. The application of claim 12, wherein the gene chip method further includes a probe for nucleic acid sequence hybridization, wherein the nucleic acid sequence is designed based on the biological precursor of the marker gene or the marker gene.
